# EUROPEAN PATENT APPLICATION

(11) **EP 4 266 053 A1**
(43) Date of publication of application: **25.10.2023**
(21) Application number: 23168226.1
(22) Date of filing: 17.04.2023
(51) Int. Cl.: G01N 33/487, G16B 40/20, B01L 3/00

(54) **COMPUTATIONAL METHOD FOR CHARACTERIZING A BIOPOLYMER PROPERTY OF A BIOPOLYMER**

(30) Priority: 19.04.2022 US 202217723939
(71) Applicant: Robert Bosch GmbH, 70442 Stuttgart (DE)
(72) Inventor: Johnson, Christopher, San Carlos, California, 94070 (US); Cunha, Carlos Eduardo, Menlo Park, CA, 94025 (US); Fomina, Nadezda, Redwood City, California, 94061 (US); Gadelrab, Karim, Boston, MA, 02111 (US); Bucci, Giovanna, Santa Clara, California, 95051 (US); Shin, Young Shik, Mountain View, California, 94040 (US)

(57) **Abstract**

A computational method for characterizing a biopolymer property of a biopolymer. The computational method includes receiving a dimensional representation of a molecule concentration over time within a fluid flow of a fluid medium flowing through a fluid channel including the biopolymer; predicting a fluid flow velocity and/or a fluid flow pressure of the fluid medium in response to the dimensional representation of the molecule concentration over time within the fluid medium using a machine learning model; and characterizing the biopolymer property of the biopolymer in response to the fluid flow velocity and/or the fluid flow pressure.

## Description

### TECHNICAL FIELD

The present disclosure relates to a computational method for characterizing a biopolymer property of a biopolymer using a machine learning model (e.g., a neural network).

### BACKGROUND

DNA is one type of biopolymer. DNA is the central storage unit of genetic information, and extracting this information is one major goal in the field of biology. Through gene sequencing, insights into genome variation, genetic mutation and replication dynamics can be achieved. Sequencing technology has made enormous strides over the past decades in reading genetic information. However, the vision of extracting the genetic code directly from a single long DNA molecule out of a single cell has still met with limited success. While human DNA has billions of base pairs, the direct read of base pair sequences is hindered by the complex compactification of DNA inside the cell. Hence, the folding and twisting of the soft condensed matter of DNA must be unraveled to access the genetic sequence.

Due to the extreme length of DNA (human chromosome of 250 million base pairs has a full stretched length of about 8.5 cm), the DNA is first sheared or enzymatically digested into fragments of a maximum length of few millimeters and incorporated into bacterial or yeast artificial chromosomes. Libraries of fragments of about 1000 base pairs can be directly sequenced and analyzed.

DNA linearization plays a crucial role in gene sequencing. Different techniques rely on hydrodynamic forces to achieve DNA extension. One attractive example is molecular combing. There, a silanized coverslip is lowered into a reservoir containing DNA. During the coverslip residence time inside the reservoir, one or both ends of DNA are attached to the coverslip surface. The coverslip is slowly withdrawn from the surface where an air-liquid meniscus is formed. The capillary forces will stretch the DNA molecule. The technique is fast and relatively simple; however, it does not allow for reliable manipulation of DNA fragments longer than a few hundred microns. There remains a need to develop processes that linearize DNA and other biopolymers so that the properties of these biopolymers may be characterized.

### SUMMARY

According to one embodiment, a computational method for characterizing a biopolymer property of a biopolymer is disclosed. The computational method includes receiving a dimensional representation of a molecule concentration over time within a fluid flow of a fluid medium flowing through a fluid channel including the biopolymer; predicting a fluid flow velocity and/or a fluid flow pressure of the fluid medium in response to the dimensional representation of the molecule concentration over time within the fluid medium using a machine learning model; and characterizing the biopolymer property of the biopolymer in response to the fluid flow velocity and/or the fluid flow pressure.

According to another embodiment, a non-transitory computer-readable medium tangibly embodying computer readable instructions for a software program is disclosed. The software program is executable by a processor of a computing device to provide the following operations: receiving a dimensional representation of a molecule concentration over time within a fluid flow of a fluid medium flowing through a fluid channel including the biopolymer; predicting a fluid flow velocity and/or a fluid flow pressure of the fluid medium in response to the dimensional representation of the molecule concentration over time within the fluid medium using a machine learning model; and characterizing the biopolymer property of the biopolymer in response to the fluid flow velocity and/or the fluid flow pressure.

According to yet another embodiment, a computer system for characterizing a biopolymer property of a biopolymer including a computer having a processor for executing computer-readable instructions and a memory for maintaining the computer-executable instructions is disclosed. The computer-executable instructions when executed by the processor perform the following functions: receiving a dimensional representation of a molecule concentration over time within a fluid flow of a fluid medium flowing through a fluid channel including the biopolymer; predicting a fluid flow velocity and/or a fluid flow pressure of the fluid medium in response to the dimensional representation of the molecule concentration over time within the fluid medium using a machine learning model; and characterizing the biopolymer property of the biopolymer in response to the fluid flow velocity and/or the fluid flow pressure.

### BRIEF DESCRIPTION OF THE DRAWINGS

Figure 1 illustrates an example process for characterizing a biopolymer property of a biopolymer.
Figure 2 illustrates a schematic representation of a fluid channel including a fluid flow of dye molecules and a biopolymer.
Figure 3 illustrates a computer system including a computing device for implementing a computational method for characterizing a biopolymer property of a biopolymer using a machine learning model (e.g., a neural network).

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. Also, unless expressly stated to the contrary: percent, "parts of," and ratio values are by weight; the term "polymer" includes "oligomer," "copolymer," "terpolymer," and the like; the description of a group or class of materials as suitable or preferred for a given purpose in connection with the invention implies that mixtures of any two or more of the members of the group or class are equally suitable or preferred; molecular weights provided for any polymers refers to number average molecular weight; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

This invention is not limited to the specific embodiments and methods described below, as specific components and/or conditions may, of course, vary. Furthermore, the terminology used herein is used only for the purpose of describing embodiments of the present invention and is not intended to be limiting in any way.

As used in the specification and the appended claims, the singular form "a," "an," and "the" comprise plural referents unless the context clearly indicates otherwise. For example, reference to a component in the singular is intended to comprise a plurality of components.

Except where expressly indicated, all numerical quantities in this description indicating dimensions or material properties are to be understood as modified by the word "about" in describing the broadest scope of the present disclosure.

The first definition of an acronym or other abbreviation applies to all subsequent uses herein of the same abbreviation and applies mutatis mutandis to normal grammatical variations of the initially defined abbreviation. Unless expressly stated to the contrary, measurement of a property is determined by the same technique as previously or later referenced for the same property.

The term "substantially" may be used herein to describe disclosed or claimed embodiments. The term "substantially" may modify a value or relative characteristic disclosed or claimed in the present disclosure. In such instances, "substantially" may signify that the value or relative characteristic it modifies is within ± 0%, 0.1%, 0.5%, 1%, 2%, 3%, 4%, 5% or 10% of the value or relative characteristic.

Reference is being made in detail to compositions, embodiments, and methods of embodiments known to the inventors. However, disclosed embodiments are merely exemplary of the present disclosure which may be embodied in various and alternative forms. Therefore, specific details disclosed herein are not to be interpreted as limiting, rather merely as representative bases for teaching one skilled in the art to variously employ the present disclosure.

A macromolecule (e.g., a biopolymer) may be threaded through a fluid channel (e.g., a micro-fluid channel or a nano-fluid channel) to elongate the macromolecule from an equilibrium coiled state to an uncoiled state, thereby providing a physical mechanism to manipulate the conformation of individual polymer chains within the macromolecule and to determine one or more properties of the macromolecule. The transport of the macromolecules through the fluid channel may be achieved by applying an electric field across the fluid channel, which may induce an electrophoretic force on the charged polymer chains of the macromolecule to pull the macromolecule into the confined region of the fluid channel. The pulling force should be large enough to overcome the free-energy barrier that results from the reduced conformational entropy of the confined macromolecule.

The sequential passage of molecular segments through a nanoscale volume may be used as a strategy for base-pair detection of genomic DNA. Electrical or optical probing of the nanoscale volume produces a highly localized signal that can be correlated to the structure or nucleotide sequence of the DNA. Increasing the electrophoretic force tends to reduce the activation energy for DNA threading, therefore increasing the system throughput. However, the electrophoretic force also controls the speed of the DNA passing through the detection volume, and large electric fields may increase the DNA translocation velocity too high to perform accurate detection and analysis. Therefore, what is needed is an active control of the DNA translocation velocity through the fine tuning of the electric field for the precise sequencing measurements.

In one or more embodiments, a computational method is disclosed to predict a fluid flow velocity and/or a fluid flow pressure in a fluid flow in a fluid channel (e.g., a micro-fluid channel or a nano-fluid channel) using a machine learning model applied to a dimensional representation (e.g., three-dimensional representation) of a molecular concentration over time within the fluid flow in the fluid channel. The fluid flow may be driven by an external electric field that cause the ions in the fluid medium (e.g., buffer solution including dye molecules) to migrate. The computational methods of one or more embodiments may be beneficial to optimize fluid channel design and/or to control the motion of a biopolymer in a fluid channel for the purpose of characterizing its properties.

The fluid flow response to the motion of a macromolecule (e.g., a biopolymer) may be indicative of several properties of the macromolecule (e.g., effective drag, mechanical stiffness, effective charge, and/or relaxation time).

In one embodiment, the machine learning model may be a physics-informed neural network model. A physics-informed neural network model may have a benefit of not requiring specific assumptions on the boundary conditions and charge equilibration of the fluid medium. The computational method may use imaging of a spatial pattern of dye molecules concentration over time. The imaging may not include tracking of each dye molecule, but instead track the dye molecules in aggregate, thereby reducing computing resources. The physics-informed neural network model used in the computational methods and systems of one or more embodiments may be significantly less computationally demanding compared to known methods and systems. In one or more embodiments, the computational method assumes that a fluid flow of the buffer solution in response to the electric field creates a distinctive optical pattern, and that a machine learning model can interpret this optical signal and provide the spatial distribution of fluid velocity and/or pressure.

In one or more embodiments, a physics-informed neural network method may be beneficial in interpreting noisy low-resolution data and/or complex geometries. However, certain physics-informed neural network methods utilize a large neural network with a fully connected architecture, which may be inefficient at handling very high dimensional datasets. To reduce the complexity and volume of the training data, one or more embodiments implement a Latin Hypercube down-sampling strategy to pick a relatively small number of training points. Alternatively, neural networks, including convolutional and recursive architectures, may be used in the machine learning model to simplify data while retaining the largest information content.

In one or more embodiments, the fluid flow velocity and/or the fluid flow pressure may be used to characterize one or more biopolymer properties of a biomolecule. The information on the fluid flow velocity and the fluid flow pressure fields may infer a desired property by a comparison with the fluid response around a biomolecule of one or more known properties. Alternatively, the fluid velocity and/or pressure fields may be input in a dynamic coarse-grained model of the macromolecule to quantify one or more desired properties.

In one or more embodiments, a computational method for characterizing a biopolymer property of a biopolymer within a fluid medium is disclosed. The computational method may rely on data driven computational tools. The computational method may also rely on imaging of a concentration of dye molecules in the fluid medium. The computational method may predict a velocity and/or a pressure characterizing a fluid flow of the fluid medium containing the biopolymer. The computational method may be employed to characterize one or more of the following biopolymer properties, including without limitation, translocation speed, mechanical stiffness, relaxation time, and effective charge. Non-limiting examples of biopolymers include DNA, RNA, microRNA, proteins, and lipids. The fluid medium may flow through a fluid channel (e.g., micro-fluid channel or nano-fluid channel).

The computational method may account for one or more of the following considerations. The flow of a buffer solution with dye molecules in response to an electric field creates an optical pattern that can be used to study micro- and nano-fluidics and its impact on biopolymer electrophoresis. The optical pattern of the diffusing dye buffer solution may be sensitive or responsive to one or more design parameters of the fluid channel (e.g., a fluid channel size, a presence of one or more pillars within the fluid channel, and/or a fluid inlet profile) and/or one or more operational conditions of the fluid flow and/or the fluid channel (e.g., a salt concentration, an electric field strength, a fluid channel wall charge and/or a fluid channel wall surface treatment). One or more embodiments of the computational method may characterize a biopolymer property of a biopolymer having biopolymer segments less than 1 micrometer long, where direct imaging of biopolymer dynamics may not be available.

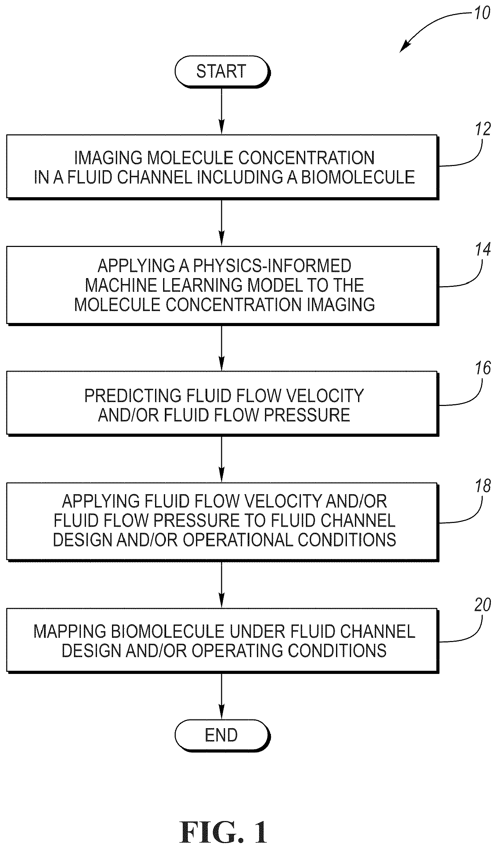

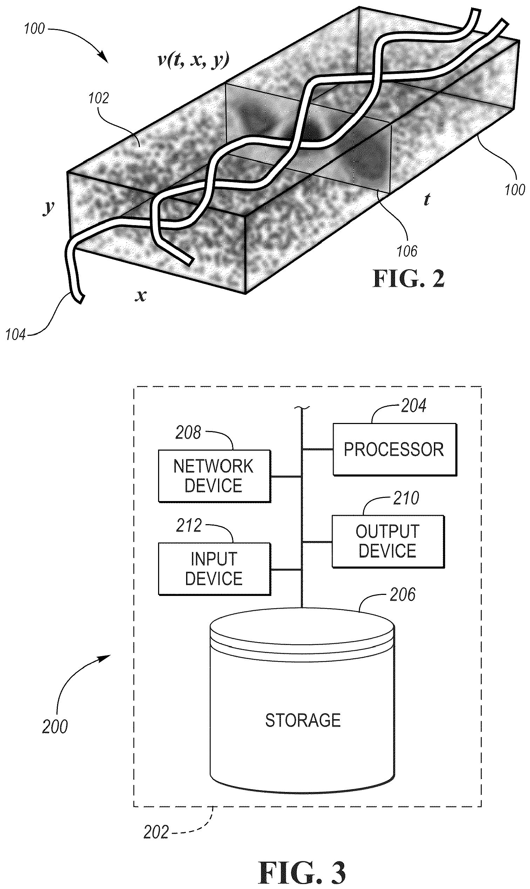

Figure 1 illustrates an example process 10 for characterizing a biopolymer property of a biopolymer. In an example, process 10 may be performed by one or more computing devices as described in further detail with respect to Figure 3.

At operation 12, a dimensional representation of a molecule concentration over time within a fluid flow of a fluid medium flowing through a fluid channel is imaged. The molecule may be a dye molecule. The fluid medium may be a buffer solution. The fluid channel may be a micro-fluid channel or a nano-fluid channel. Figure 2 illustrates a schematic representation of fluid channel 100 including a fluid flow of dye molecules 102 and around biopolymer 104. Biopolymer 104 may be an unlabeled DNA. As depicted by image 106 in Figure 2, the concentration of dye molecules 102 is imaged in the t, x, and y dimensions of fluid channel 100. Two or more dye molecules having different diffusive properties may be used simultaneously to enhance the information contained in the concentration pattern. For example, the dye molecules may include first and second dye molecule types where the first dye molecule type has a first diffusion property, and the second dye molecule type has a second diffusion property. While biopolymer 104 is not labelled, its location and transport dynamics through fluid channel 100 is detected through perturbations induced to the fluid flow. In one or more embodiments using operation 12, the coupling between biomolecule electrophoresis, confinement, and electroosmotic flow directly from imaging may be examined, and may be used to improve fluid channel design. Reducing the fluid channel size and/or using a buffer solution with relatively low ionic strength may contribute to increasing the coupling between biomolecule dynamics and fluid flow.

At operation 14, a physics-informed machine learning model is applied to the molecule concentration imaging. The physics-informed machine learning model may use a Latin Hypercube down-sampling strategy to pick a relatively small number of training points. In other embodiments, a different machine learning model may be used. The machine learning model may quantify the effective charge of a biomolecule in a fluid medium (e.g., buffer solution) using the imaging provided by operation 12. The machine learning model may characterize a relationship of a translocation velocity of a biopolymer to a geometry of the fluid channel using the imaging provided by operation 12. The machine learning model may characterize a relationship of the translocation velocity of the biopolymer to one or more surface treatments or wall charges on the fluid channel using the imaging provided by operation 12. The machine learning model may characterize one or more of the following biopolymer properties: elastic response, relaxation time, and/or conformation. The conformation of biopolymers may include the presence of defects in the molecule elongation such as folds, hairpin, and knots. The defect may be detected through the perturbation induced to the fluid flow using the set up shown in Figure 2 and described in operation 12.

At operation 16, the fluid flow velocity and/or the fluid flow pressure is predicted using the dimensional representation of molecule concentration over time using the physics-informed learning model.

At operation 18, the fluid flow velocity and/or the fluid flow pressure are applied to fluid channel design parameters and/or operational conditions. Non-limiting examples of fluid channel design parameters include fluid channel size, presence of one or more pillars within the fluid channel, and/or fluid inlet profile. Non-limiting examples of operational conditions include salt concentration, electric field strength, fluid channel wall charge and/or fluid channel wall surface treatment.

At operation 20, a biomolecule can be mapped under the channel design parameters and/or operational conditions. In one or more embodiments, the design and range of operation of the fluid channel is optimized to achieve efficient and accurate biomolecule mapping and/or sequencing.

Figure 3 illustrates computer system 200 including computing device 202 for implementing a computational method for characterizing a biopolymer property of a biopolymer using a machine learning model. Referring to Figure 3, computing device 202 may be the hardware performing the operations set forth in Figure 1. As shown, computing device 202 may include processor 204 that is operatively connected to storage 206, network device 208, output device 210, and input device 212. In other embodiments, computing device 202 may have more, fewer, or different components than shown in Figure 3.

Processor 204 may include one or more integrated circuits that implement the functionality of a central processing unit (CPU) and/or graphics processing unit (GPU). In some examples, processors 204 are a system on a chip (SoC) that integrates the functionality of the CPU and GPU. The SoC may optionally include other components such as, for example, storage 206 and the network device 208 into a single integrated device. In other examples, the CPU and GPU are connected to each other via a peripheral connection device such as Peripheral Component Interconnect (PCI) express or another suitable peripheral data connection. In one example, the CPU is a commercially available central processing device that implements an instruction set such as one of the x86, ARM, Power, or microprocessor without interlocked pipeline stage (MIPS) instruction set families. In some examples, a neural processing unit (NPU) may be applied, *e.g.*, if pretrained machine learning models are being used.

Regardless of the specifics, during operation processor 204 executes stored program instructions that are retrieved from storage 206. The stored program instructions, accordingly, include software that controls the operation of processors 204 to perform the operations described herein. Storage 206 may include both non-volatile memory and volatile memory devices. The non-volatile memory includes solid-state memories, such as negative-AND (NAND) flash memory, magnetic and optical storage media, or any other suitable data storage device that retains data when system 200 is deactivated or loses electrical power. The volatile memory includes static and dynamic random-access memory (RAM) that stores program instructions and data during operation of system 200.

The GPU may include hardware and software for display of at least 2D and optionally 3D graphics to output device 210. Output device 210 may include a graphical or visual display device, such as an electronic display screen, projector, printer, or any other suitable device that reproduces a graphical display. As another example, output device 210 may include an audio device, such as a loudspeaker or headphone. As yet a further example, output device 210 may include a tactile device, such as a mechanically raiseable device that may, in an example, be configured to display braille or another physical output that may be touched to provide information to a user.

Input device 212 may include any of various devices that enable the computing device 202 to receive control input from users. Examples of suitable input devices that receive human interface inputs may include keyboards, mice, trackballs, touchscreens, voice input devices, graphics tablets, and the like.

Network devices 208 may each include any of various devices that enable computing device 202 to send and/or receive data from external devices over networks. Examples of suitable network devices 208 include an Ethernet interface, a Wi-Fi transceiver, a cellular transceiver, or a BLUETOOTH or BLUETOOTH Low Energy (BLE) transceiver, or other network adapter or peripheral interconnection device that receives data from another computer or external data storage device, which can be useful for receiving large sets of data in an efficient manner.

The processes, methods, or algorithms disclosed herein can be deliverable to/implemented by a processing device, controller, or computer, which can include any existing programmable electronic control unit or dedicated electronic control unit. Similarly, the processes, methods, or algorithms can be stored as data and instructions executable by a controller or computer in many forms including, but not limited to, information permanently stored on non-writable storage media such as read-only memory (ROM) devices and information alterably stored on writeable storage media such as floppy disks, magnetic tapes, compact discs (CDs), RAM devices, and other magnetic and optical media. The processes, methods, or algorithms can also be implemented in a software executable object. Alternatively, the processes, methods, or algorithms can be embodied in whole or in part using suitable hardware components, such as Application Specific Integrated Circuits (ASICs), Field-Programmable Gate Arrays (FPGAs), state machines, controllers or other hardware components or devices, or a combination of hardware, software and firmware components.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

## Claims

1. A computational method for characterizing a biopolymer property of a biopolymer, the method comprising:
receiving a dimensional representation of a molecule concentration over time within a fluid flow of a fluid medium flowing through a fluid channel including the biopolymer;
predicting a fluid flow velocity and/or a fluid flow pressure of the fluid medium in response to the dimensional representation of the molecule concentration over time within the fluid medium using a machine learning model; and
characterizing the biopolymer property of the biopolymer in response to the fluid flow velocity and/or the fluid flow pressure.

2. The computational method of claim 1, wherein the fluid medium is a buffer solution including dye molecules, the molecule concentration over time is a concentration over time of the dye molecules, and the dimensional representation is an optical pattern.

3. The computational method of claim 1, wherein the fluid channel is a biopolymer mapping device.

4. The computational method of claim 1, wherein the fluid channel is a micro-fluid channel or a nano-fluid channel.

5. The computational method of claim 1, wherein the dimensional representation is responsive to one or more design parameters of the fluid channel and/or one or more operational conditions of the fluid flow and/or the fluid channel.

6. The computational method of claim 5, wherein the one or more design parameters of the fluid channel include a fluid channel size, a presence of one or more pillars within the fluid channel and/or a fluid inlet profile.

7. The computational method of claim 5, wherein the one or more operational conditions of the fluid flow and/or the fluid channel include a salt concentration, an electric field strength, a fluid channel wall charge and/or a fluid channel wall surface treatment.

8. The computational method of claim 1, wherein the biopolymer includes biopolymer segments having lengths of less than 1 micron.

9. The computational method of claim 1, wherein the machine learning model is a physics-informed neural network model.

10. The computational method of claim 1, wherein the biopolymer property includes a translocation speed, an effective drag, an elastic response, a conformation, a mechanical stiffness, a relaxation time, and/or an effective charge.

11. The computational method of claim 1, wherein the biopolymer is DNA, RNA, microRNA, a protein, or a lipid.

12. The computational method of claim 1, wherein the fluid medium is a buffer solution including dye molecules, the molecular concentration over time is a concentration over time of the dye molecules, the dye molecules include a first dye molecule type having a first diffusion property and a second dye molecule type having a second dye molecule type having a second diffusion property different than the first diffusion property.

13. The computational method of claim 1, wherein the biopolymer property includes an effective charge of the biopolymer and/or a biopolymer translocation velocity.

14. The computational method of claim 1, further comprising controlling a motion of the biopolymer in the fluid channel depending on the biopolymer property of the biopolymer.

15. The computational method of claim 14, further comprising characterizing a relationship between the biopolymer translocation velocity and one or more design parameters of the fluid channel and/or one or more operational conditions of the fluid flow and/or the fluid channel.

16. The computational method of claim 1, further comprising driving the fluid flow by an external electric field causing migration of ions in the fluid medium.

17. A non-transitory computer-readable medium tangibly embodying computer readable instructions for a software program, the software program being executable by a processor of a computing device to provide operations comprising:
receiving a dimensional representation of a molecule concentration over time within a fluid flow of a fluid medium flowing through a fluid channel including a biopolymer;
predicting a fluid flow velocity and/or a fluid flow pressure of the fluid medium in response to the dimensional representation of the molecule concentration over time within the fluid medium using a machine learning model; and
characterizing a biopolymer property of the biopolymer in response to the fluid flow velocity and/or the fluid flow pressure.

18. The non-transitory computer-readable medium of claim 17, wherein the fluid medium is a buffer solution including dye molecules, the molecule concentration over time is a concentration over time of the dye molecules, and the dimensional representation is an optical pattern.

19. A computer system for characterizing a biopolymer property of a biopolymer including a computer having a processor for executing computer-readable instructions and a memory for maintaining the computer-executable instructions, the computer-executable instructions when executed by the processor perform the following functions:
receiving a dimensional representation of a molecule concentration over time within a fluid flow of a fluid medium flowing through a fluid channel including the biopolymer;
predicting a fluid flow velocity and/or a fluid flow pressure of the fluid medium in response to the dimensional representation of the molecule concentration over time within the fluid medium using a machine learning model; and
characterizing the biopolymer property of the biopolymer in response to the fluid flow velocity and/or the fluid flow pressure.

20. The computer system of claim 19, wherein the fluid medium is a buffer solution including dye molecules, the molecule concentration over time is concentration over time of the dye molecules, and the dimensional representation is an optical pattern.
